# FASCICULE DE BREVET EUROPEEN

(11) **EP 2 603 602 B1**
(45) Date de publication et mention de la délivrance du brevet: **27.08.2014**
(21) Numéro de dépôt: 11754519.4
(22) Date de dépôt: 10.08.2011
(51) Int. Cl.: C12Q 1/48, G01N 33/68

(54) **Méthode de diagnostic des maladies neurodégénératives**
Verfahren zur Diagnose von neurodegenerativen Erkrankungen
Method for diagnosing neurodegenerative diseases

(30) Priorité: 11.08.2010 FR 1003337
(43) Date de publication de la demande: 19.06.2013
(73) Titulaire: Assistance Publique Hôpitaux De Paris, 75004 Paris 4 (FR)
(72) Inventeur: HUGON, Jacques, F-60500 Chantilly (FR); PAQUET, Claire, F-60500 Chantilly (FR); DUMURGIER, Julien, F-75015 Paris (FR); MOUTON-LIGER, François, F-94800 Villejuif (FR)
(74) Mandataire: Vial, Lionel
(86) Numéro de dépôt international: PCT/IB2011/053571
(87) Numéro de publication internationale: WO 2012/020385

(56) Documents cités:
- PACCALIN MARC ET AL: "Activated mTOR and PKR Kinases in Lymphocytes Correlate with Memory and Cognitive Decline in Alzheimer's Disease", DEMENTIA AND GERIATRIC COGNITIVE DISORDERS, KARGER, BASEL, CH, vol. 22, no. 4, 1 janvier 2006 (2006-01-01), pages 320-326, XP008121317, ISSN: 1420-8008, DOI: DOI:10.1159/000095562
- PEEL ALYSON L ET AL: "Activation of the cell stress kinase PKR in Alzheimer's disease and human amyloid precursor protein transgenic mice.", NEUROBIOLOGY OF DISEASE OCT 2003 LNKD- PUBMED:13678666, vol. 14, no. 1, octobre 2003 (2003-10), pages 52-62, XP002612081, ISSN: 0969-9961
- PAGE G ET AL: "Activated double-stranded RNA-dependent protein kinase and neuronal death in models of Alzheimer's disease", NEUROSCIENCE, NEW YORK, NY, US, vol. 139, no. 4, 1 janvier 2006 (2006-01-01), pages 1343-1354, XP024986702, ISSN: 0306-4522, DOI: DOI:10.1016/J.NEUROSCIENCE.2006.01.047 [extrait le 2006-01-01]
- HAMPEL HARALD ET AL: "Advances in the development of biomarkers for Alzheimer's disease: from CSF total tau and Abeta(1-42) proteins to phosphorylated tau protein.", BRAIN RESEARCH BULLETIN 15 AUG 2003 LNKD- PUBMED:12909294, vol. 61, no. 3, 15 août 2003 (2003-08-15), pages 243-253, XP002612079, ISSN: 0361-9230
- MATTSSON NIKLAS ET AL: "Alzheimer's disease and CSF biomarkers: key challenges for broad clinical applications.", BIOMARKERS IN MEDICINE DEC 2009 LNKD- PUBMED:20477711, vol. 3, no. 6, décembre 2009 (2009-12), pages 735-737, XP002612080, ISSN: 1752-0371
- JACQUES HUGON ET AL: "Could PKR inhibition modulate human neurodegeneration?", EXPERT REVIEW OF NEUROTHERAPEUTICS, vol. 9, no. 10, 1 October 2009 (2009-10-01), pages 1455-1457, XP055096931, ISSN: 1473-7175, DOI: 10.1586/ern.09.92

## Description

### Domaine de l'invention

La présente invention concerne une méthode de diagnostic des maladies neurodégénératives, notamment la maladie d'Alzheimer.

### Arrière-plan technique

Le diagnostic des maladies neurodégénératives n'est pas aisé. Ainsi, pour la maladie d'Alzheimer, selon les critères classiques dits NINCDS-ADRDA (McKhann et al. (1984) Neurology 34:939-944), on distingue entre maladie d'Alzheimer possible, probable et certaine, le diagnostic d'une maladie d'Alzheimer certaine ne pouvant être établi que *post-mortem,* après autopsie du malade et examen neuropathologique du cerveau mettant en évidence l'existence de plaques séniles d'une part, et une dégénérescence neurofibrillaire d'autre part.

Le diagnostic d'une maladie d'Alzheimer possible ou probable repose essentiellement sur des critères cliniques et des tests neuropsychologiques qui visent tout d'abord à établir si un individu présente un syndrome démentiel, en général selon les critères du DSM IV *(*Diagnostic and Statistical Manual for Mental Disorders, 4ème édition, American Psychiatric Association, 1994), puis à déterminer l'étiologie de la démence. Les critères de démences comprennent essentiellement l'association d'un trouble de la mémoire et d'autres fonctions cognitives retentissant sur les activités socio-professionnelles et entraînant un déclin par rapport au fonctionnement antérieur. Le diagnostic se renforce par le suivi de l'évolution du patient, ce qui permet de préciser les causes de la démence, notamment en écartant d'autres causes de déclin cognitif. A ce titre, l'imagerie cérébrale est un élément important du diagnostic en évaluant l'atrophie régionale hippocampique, qui peut toutefois être présente dans d'autres maladies atteignant les personnes âgées, et en excluant d'autres causes de troubles cognitifs, telles que des séquelles d'accidents vasculaires cérébraux.

Cependant, on considère généralement qu'environ seulement 85% des cas de maladie d'Alzheimer probable sont confirmés *post-mortem.* Les 15% de faux positifs résultent généralement d'autres maladies neurodégénératives, telles que les démences frontotemporales, dont notamment la démence avec corps de Lewy (Delacourte (1998) Annales de Biologie Clinique 56:133-142). La méthodologie actuelle de diagnostic de la maladie d'Alzheimer manque donc de spécificité. En outre, il apparaît qu'entre le déclenchement de la maladie, défini par l'apparition des premiers troubles cognitifs, et les premiers symptômes de démence pouvant donner lieu à l'établissement d'un diagnostic de maladie d'Alzheimer possible ou probable, il peut s'étendre une période de plus de 10 ans (Amieva et al. (2008) Ann. Neurol. 64:479-480). Le diagnostic de la maladie d'Alzheimer est donc actuellement établi tardivement, alors que la maladie se trouve à un stade très avancé. Les traitements actuellement envisagés pour la maladie d'Alzheimer nécessiteraient toutefois, pour être efficace, d'être mis en oeuvre dès le début du processus pathologique, par exemple alors que l'individu à traiter ne présente encore que des troubles cognitifs légers *(Mild Cognitive Impairement,* MCI), ce qui implique de pouvoir précocement diagnostiquer la maladie.

De fait, des examens complémentaires, tels que le dosage de marqueurs biochimiques dans le liquide céphalorachidien de patients, peuvent être effectués pour améliorer le diagnostic de la maladie d'Alzheimer. Ainsi, il a notamment été montré que la détermination des niveaux, dans le liquide céphalorachidien, du peptide β-amyloïde₁₋₄₂ (Aβ42) et du niveau total du peptide tau *(Tubulin Associated Unit),* c'est-à-dire à la fois sous forme phosphorylée et sous forme non phosphorylée, permettait de diagnostiquer la maladie d'Alzheimer avec une sensibilité de 92% et une spécificité de 89% (Sunderland et al. (2003) JAMA 289:2094-2103). Depuis, un troisième marqueur a été ajouté, en plus de Aβ42 et de tau total (T-tau), à savoir tau phosphorylée (ptau), notamment au niveau de la thréonine 181 (ptau181). En outre, il a pu être montré que la combinaison de ces trois marqueurs permettait de détecter une maladie d'Alzheimer naissante chez des individus souffrant de MCI avec une sensibilité de 83% et une spécificité de 72% (Mattson et al. (2009) JAMA 302:385-393).

Toutefois, à l'heure actuelle, ces dosages ne sont pas réalisés en routine, en partie car le gain qu'ils permettent en terme fiabilité et de précocité du diagnostic de la maladie d'Alzheimer n'est pas suffisant pour justifier d'avoir recours à ces derniers systématiquement.

La protéine kinase ARN double-brin dépendante (PKR) est une sérine/thréonine kinase dont la principale cible est le facteur d'initiation de la traduction eukaryote 2α (eIF2α). PKR existe sous une forme activée par phosphorylation de la thréonine en position 446 et/ou de la thréonine en position 451. Des niveaux élevés de PKR activée ont pu être notamment mis en évidence dans le cerveau de patients souffrant de la maladie d'Alzheimer, de la maladie de Huntington, et de la maladie de Creutzfeldt-Jakob, ainsi que dans les lymphocytes sanguins de patients souffrant de la maladie d'Alzheimer (pour revue voir Hugon et al. (2009) Expert Rev. Neurother. 9:1455-1457).

### Description de l'invention

L'invention découle de la mise en évidence, par les présents inventeurs, que la détermination du niveau de PKR, notamment sous sa forme activée phosphorylée, dans le liquide céphalorachidien d'individus est utile pour diagnostiquer la maladie d'Alzheimer chez ces individus de manière sélective et spécifique, y compris à un stade naissant ou précoce, éventuellement asymptomatique, de la maladie.

Ainsi, la présente invention concerne une méthode de diagnostic, *notamment in vitro,* d'une maladie neurodégénérative chez un individu, dans laquelle :
- on détermine le niveau de la protéine kinase ARN double-brin dépendante (PKR) dans un échantillon biologique de l'individu, notamment un échantillon de liquide céphalorachidien,
- on en déduit si l'individu souffre d'une maladie neurodégénérative.

La présente invention concerne également une méthode de diagnostic, *notamment in vitro,* d'une maladie neurodégénérative chez un individu, dans laquelle :
- on détermine le rapport du niveau de PKR activée sur le niveau total de PKR dans un échantillon biologique de l'individu, notamment un échantillon de liquide céphalorachidien,
- on en déduit si l'individu souffre d'une maladie neurodégénérative.

La présente invention concerne également une méthode de diagnostic, *notamment in vitro,* d'une maladie neurodégénérative chez un individu ne présentant pas de symptôme de démence, dans laquelle :
- on détermine le niveau de la protéine kinase ARN double-brin dépendante (PKR) dans un échantillon biologique de l'individu, notamment un échantillon de liquide céphalorachidien,
- on en déduit si l'individu souffre d'une maladie neurodégénérative.

La présente invention concerne également une méthode, notamment *in vitro,* de détermination du risque qu'a un individu ne présentant pas de symptôme d'une maladie neurodégénérative, de déclarer la maladie neurodégénérative, dans laquelle :
- on détermine le niveau de la protéine kinase ARN double-brin dépendante (PKR) dans un échantillon biologique de l'individu, notamment un échantillon de liquide céphalorachidien,
- on en déduit si l'individu est à risque de développer la maladie neurodégénérative.

Comme on l'entend ici, l'expression « maladie neurodégénérative » désigne une maladie dégénérative, c'est-à-dire à dégradation progressive, affectant le système nerveux et en particulier le cerveau.

De manière préférée la maladie neurodégénérative selon l'invention est une démence. Les démences sont bien connues de l'homme du métier ; on considère généralement qu'une démence se caractérise par une détérioration progressivement du fonctionnement intellectuel d'un individu, compromettant ainsi ses facultés d'adaptation à son environnement, en particulier face à des situations nouvelles, ce qui conduit à une perte de son autonomie. De préférence, les symptômes de démence selon l'invention sont en particulier ceux définis dans le Diagnostic and Statistical Martual for Mental Disorders, 4ème édition (1994) de l'American Psychiatric Association (DSM IV) pour les démences de type maladie d'Alzheimer, à savoir :
A. Le développement de déficits cognitifs multiples se manifestant à la fois par :
   (1) des troubles de la mémoire (capacité réduite d'apprendre de nouvelles informations ou de se rappeler des informations précédemment apprises).
   (2) un (ou plusieurs) des troubles cognitifs suivants :
      (a) aphasie (trouble du langage)
      (b) apraxie (capacité réduite de mettre en oeuvre des activités motrices malgré des fonctions motrices intactes)
      (c) agnosie (incapacité à reconnaître ou à identifier des objets malgré des fonctions sensorielles intactes)
      (d) troubles du fonctionnement exécutif (c'est-à-dire planifier, organiser, fractionner en séquence, penser de manière abstraite)
B. Les déficits cognitifs des critères A1 et A2 causent chacun des troubles significatifs du fonctionnement social ou professionnel et représentent un déclin significatif par rapport à un niveau de fonctionnement antérieur.
C. L'évolution est caractérisée par un déclenchement progressif et un déclin cognitif continu.
D. Les déficits cognitifs des critères A1 et A2 ne sont dus à aucun de ce qui suit :
   (1) autres maladies du système nerveux causant des déficits progressifs cognitifs et de la mémoire (par exemple, maladie cérébrovasculaire, maladie de Parkinson, maladie de Huntington, hématome sous-dural, hydrocéphalie à pression normale, tumeur du cerveau).
   (2) maladies systémiques connues pour causer des démences (par exemple, hypothyroïdisme, carence en vitamine B12 ou en acide folique, carence en niacine, hypercalcémie, neurosyphilis, infection par le HIV).
   (3) maladies induites par des substances.
E. Les déficits ne se produisent pas uniquement au cours d'un délirium.
F. Le dérangement ne peut être mieux compris comme étant dû par un autre trouble d'Axe I (au sens du DSM IV, c'est-à-dire nécessitant une attention immédiate) (par exemple un trouble dépressif majeur, ou une schizophrénie).

De manière préférée, la maladie neurodégénérative selon l'invention est sélectionnée dans le groupe constitué de la maladie d'Alzheimer, de la maladie de Huntington, de la maladie de Creutzfeld-Jakob, et de la maladie de Parkinson.

De manière particulièrement préférée, la maladie neurodégénérative selon l'invention est la maladie d'Alzheimer.

L'individu selon l'invention est de préférence un humain. L'individu selon l'invention peut présenter un ou plusieurs symptômes de démence ou être atteint de démence.

L'individu selon l'invention peut également ne pas être atteint de démence ou ne pas présenter de symptôme de démence. Lorsqu'il ne présente pas de démence, l'individu selon l'invention peut être atteint de troubles cognitifs, notamment de troubles cognitifs légers, correspondant à la dénomination anglo-saxonne de *Mild Cognitive Impairement* (MCI), bien connue de l'homme du métier et notamment définie par Petersen et al. (1999) Arch Neurol 56:303-308. Un individu est généralement défini comme présentant un MCI en cas de plainte subjective associée à une évidence objective d'un déficit de la performance mnésique avec épargne du fonctionnement cognitif et intellectuel global et intégrité des activités de la vie de tous les jours. De préférence, un individu présentant un MCI selon l'invention a un score au test *Mini Mental State Examination* (MMSE), notamment dans la version consensuelle du Groupe de Réflexion sur les Evaluations Cognitives (GRECO), supérieur au score correspondant au 5^{ème} centile, en fonction de son âge et de son niveau socio-culturel. Par ailleurs, l'individu selon l'invention peut également ne pas présenter de troubles cognitifs.

Lorsque l'individu selon l'invention n'est pas atteint d'une démence, notamment de la maladie d'Alzheimer, ou ne présente pas de symptôme de démence, notamment de la maladie d'Alzheimer, en particulier notamment lorsqu'il présente un MCI, les méthodes selon l'invention permettent en particulier de déterminer si l'individu présente la démence, notamment la maladie d'Alzheimer, à un stade naissant ou précoce, encore asymptomatique, ou si l'individu est à risque de développer la démence, notamment la maladie d'Alzheimer.

De préférence également, le niveau, dans un échantillon de liquide céphalorachidien de l'individu, d'au moins un marqueur biologique de maladie neurodégénérative est normal. Comme on l'entend ici, le niveau d'un marqueur biologique de maladie neurodégénérative est dit normal lorsque son niveau est inférieur à la valeur seuil généralement retenue par l'homme du métier pour diagnostiquer l'individu comme étant atteint de la maladie neurodégénérative à laquelle le marqueur est associé. De nombreux marqueurs biologiques de maladies neurodégénératives, notamment de la maladie d'Alzheimer, sont connus de l'homme du métier. De préférence, le marqueur biologique de maladie neurodégénérative selon l'invention est un marqueur biologique de la maladie d'Alzheimer, notamment choisi dans le groupe constitué du marqueur β-amyloïde (Aβ), en particulier β-amyloïde₁₋₄₂ (Aβ42), de tau total (T-tau), et de tau phosphorylé (ptau), en particulier au niveau de la thréonine 181 (ptau181). Ces marqueurs et les méthodes de mesure de leur niveau dans un échantillon de liquide céphalorachidien sont bien connus de l'homme du métier et sont notamment définis dans Sunderland et al. (2003) JAMA 289:2094-2103, Blennow et al. (2006) Lancet 368:387-403 et dans Mattson et al. (2009) JAMA 302:385-393.

La protéine kinase ARN double-brin dépendante (PKR) également nommée E2AK2 est bien connue de l'homme du métier et est notamment définie dans Hugon et al. (2009) Expert Rev. Neurother. 9:1455-1457 ou par la référence d'accès P19525 de la base de données UniProtKB. Comme on l'entend ici, l'expression « protéine kinase ARN double-brin dépendante » ou « PKR » se réfère indifféremment à PKR non phosphorylée, à PKR phosphorylée (pPKR) sur la thréonine position 446 (pPKR446) et/ou sur la thréonine en position 451 (pPKR451), ou à l'ensemble de ces formes de PKR. L'ensemble des formes de PKR dans l'échantillon biologique de l'invention, phosphorylées et non phosphorylées est également nommé PKR total.

Ainsi, dans un mode de mise en oeuvre préféré de la méthode de diagnostic ou de détermination du risque de l'invention, on déduit que l'individu souffre d'une maladie neurodégénérative, notamment de la maladie d'Alzheimer, à partir du niveau de PKR phosphorylée, notamment pPKR446, dans l'échantillon, du niveau de PKR total dans l'échantillon, ou du rapport du niveau de PKR phosphorylée, notamment pPKR446, dans l'échantillon sur le niveau de PKR total dans l'échantillon. Dans un autre mode de mise en oeuvre préféré de la méthode de diagnostic ou de détermination du risque de l'invention, on déduit que l'individu souffre d'une maladie neurodégénérative, notamment de la maladie d'Alzheimer, à partir de la comparaison entre :
- le niveau de PKR phosphorylée, notamment pPKR446, dans l'échantillon, le niveau de PKR total dans l'échantillon, ou le rapport du niveau de PKR phosphorylée, notamment pPKR446, dans l'échantillon sur le niveau de PKR total dans l'échantillon, et
- au moins une valeur prédéfinie, notamment lorsque le niveau de PKR phosphorylée, notamment pPKR446, dans l'échantillon, le niveau de PKR total dans l'échantillon, ou le rapport du niveau de PKR phosphorylée, notamment pPKR446, dans l'échantillon sur le niveau de PKR total dans l'échantillon est supérieur à la valeur prédéfinie.

La valeur prédéfinie selon l'invention peut être déterminée de manière aisée par l'homme du métier. Il peut notamment s'agir du niveau moyen de PKR selon l'invention, ou d'un multiple supérieur à 1 de ce niveau moyen, dans des échantillons de LCR d'individus témoins ne souffrant pas de troubles cognitifs. Il peut également s'agir d'une valeur seuil obtenue à partir de courbes *Receiving Operating Characteristics* (ROC) établie pour PKR total, PKR phosphorylée, notamment pPKR446, ou le rapport du niveau de PKR phosphorylée, notamment pPKR446, dans l'échantillon sur le niveau de PKR total dans l'échantillon, en fixant des valeurs particulière de sélectivité et de spécificité, notamment pour maximiser la somme de la sélectivité et de la spécificité, comme cela est illustré dans les exemples qui suivent. Ainsi, à titre d'exemple, selon l'invention un individu sera diagnostiqué comme ayant la maladie d'Alzheimer lorsque le rapport du niveau de PKR phosphorylée, notamment pPKR446, dans l'échantillon sur le niveau de PKR total dans l'échantillon, sera supérieur à 0,76 ou 0,77.

Par ailleurs, dans un autre mode de réalisation particulier, la méthode de diagnostic ou de détermination du risque selon l'invention comprend en outre la détermination du niveau d'au moins un autre marqueur biologique de maladie neurodégénérative, notamment de la maladie d'Alzheimer, tel que défini ci-dessus.

L'échantillon biologique selon l'invention est de préférence un échantillon biologique fluide, plus préférablement du liquide céphalorachidien (LCR). L'homme du métier sait bien comment obtenir du LCR à partir d'un individu, par exemple par ponction lombaire.

De préférence, dans la méthode selon l'invention, on détermine le niveau de PKR selon l'invention dans l'échantillon biologique selon l'invention par une méthode immunologique, c'est-à-dire une méthode mettant en oeuvre des anticorps, notamment monoclonaux, des fragments d'anticorps comprenant la partie de l'anticorps se liant spécifiquement à l'antigène, ou encore des aptamères, spécifiquement dirigé contre PKR, c'est-à-dire ne se liant essentiellement pas à d'autre constituant de l'échantillon biologique, notamment de l'échantillon de LCR. Comme cela apparaîtra clairement à l'homme du métier des anticorps utiles pour mesurer le niveau de PKR total sont dirigés contre une partie de PKR non modifiée par phosphorylation ; *a contrario,* des anticorps utiles pour mesurer le niveau de pPKR446 ou pPKR451 se lient sur la partie de PKR phosphorylée (et non sur la même partie lorsqu'elle n'est pas phosphorylée). De tels anticorps anti-PKR sont bien connus de l'homme du métier. Les méthodes immunologiques selon l'invention sont bien connues de l'homme du métier et recouvrent notamment le marquage de Western ou la technique ELISA.

L'invention sera davantage illustrée de manière non-limitative à l'aide des figures et des exemples qui suivent.

### Description des figures

Figures 1, 2 et 3

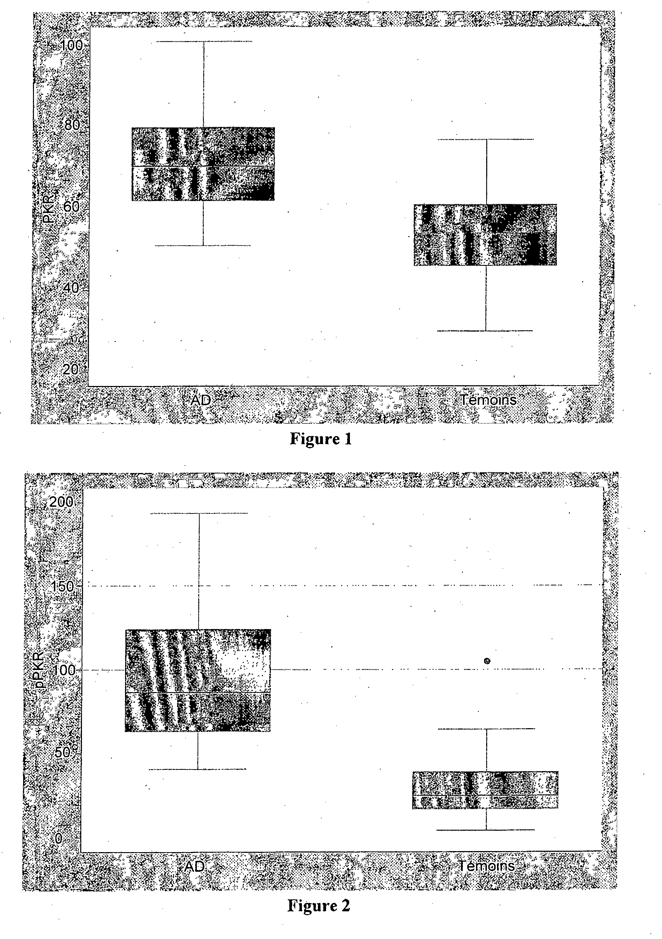

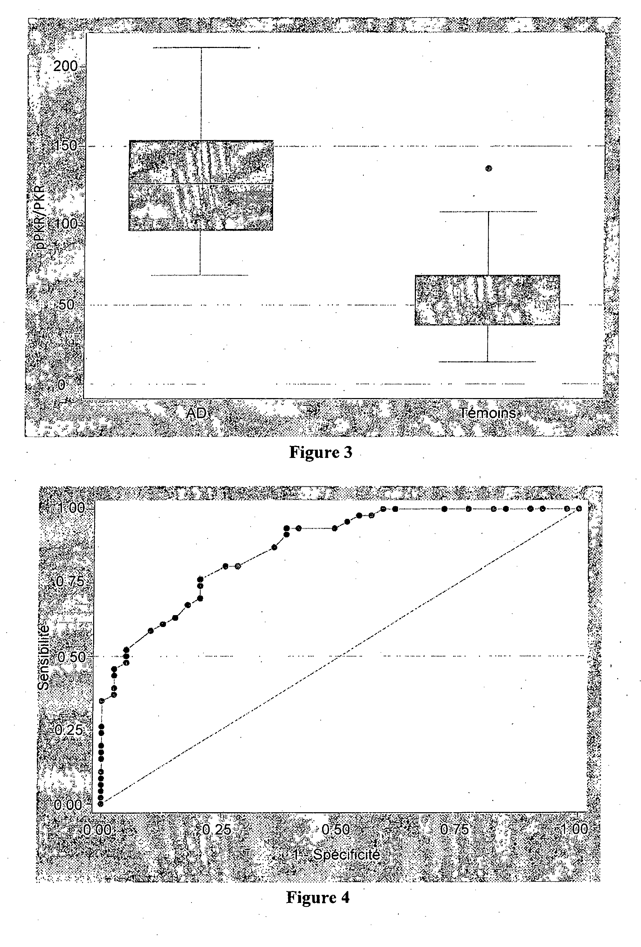

   Les figures 1 à 3 donnent des représentations en boites à moustaches des niveaux (unités arbitraires), mesurés dans le LCR de patients atteints de maladie d'Alzheimer (AD), de PKR total (PKR) (figure 1), de pPKR446 (pPKR) (figure 2) et du rapport pPKR446/PKR total multiplié par 100 (pPKR/PKR) (figure 3), par rapport à ceux mesurés dans le LCR d'individus témoins ne souffrant pas de troubles cognitifs (témoin).
Figures 4, 5 et 6

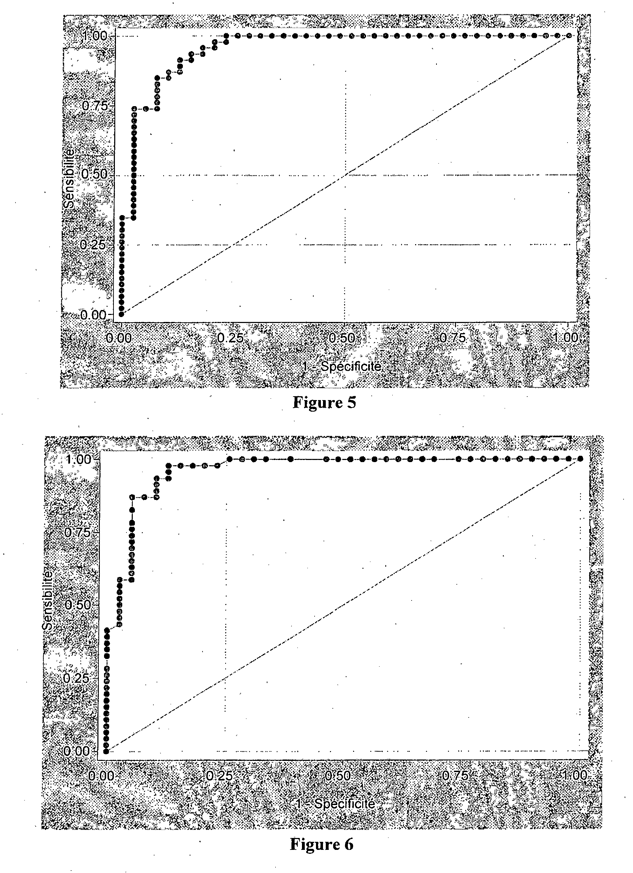

   Les figures 4 à 6 représentent les courbes ROC donnant la sélectivité (axe des ordonnées) et 1 - spécificité (axe des ordonnées) du diagnostic de la maladie d'Alzheimer à partir du niveau de PKR total (figure 4), de pPKR446 (figure 5) et du rapport pPKR446/PKR total (figure 6).
Figure 7

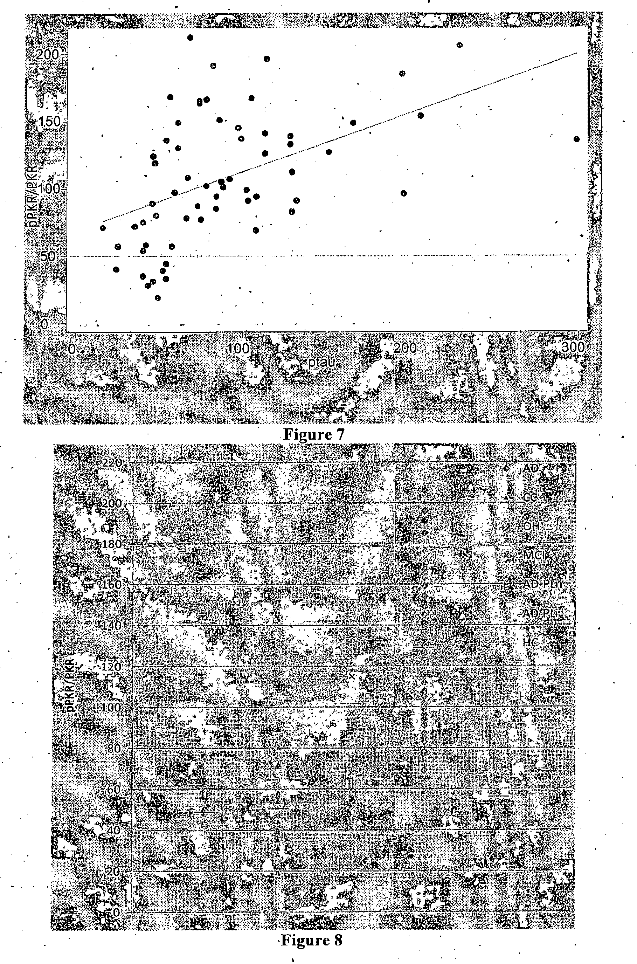

   La figure 7 représente la relation entre le rapport pPKR446/PKR total multiplié par 100 (pPKR/PKR, axe des ordonnées, unités arbitraires) et le niveau de ptau181 (ptau, axe des abscisses, pg/mL) dans le LCR ainsi que la droite de corrélation correspondante.
Figure 8
   La figure 8 représente le rapport du niveau de pPKR446 sur le niveau de PKR total (axe des ordonnées, unités arbitraires) dans le LCR de patients atteints de la maladie d'Alzheimer (AD), notamment avec des niveaux des marqueurs Aβ42, T-tau et ptau181 normaux (AD-PLn) ou intermédiaires (AD-PLi) ; d'individus témoins sans troubles cognitifs (HC) ; de témoins présentant des troubles cognitifs sans rapport avec la maladie d'Alzheimer (CC) ; d'individus présentant une démence alcoolique (OH) ; et d'individu présentant un trouble cognitif léger (MCI).
Figures 9, 10, 11

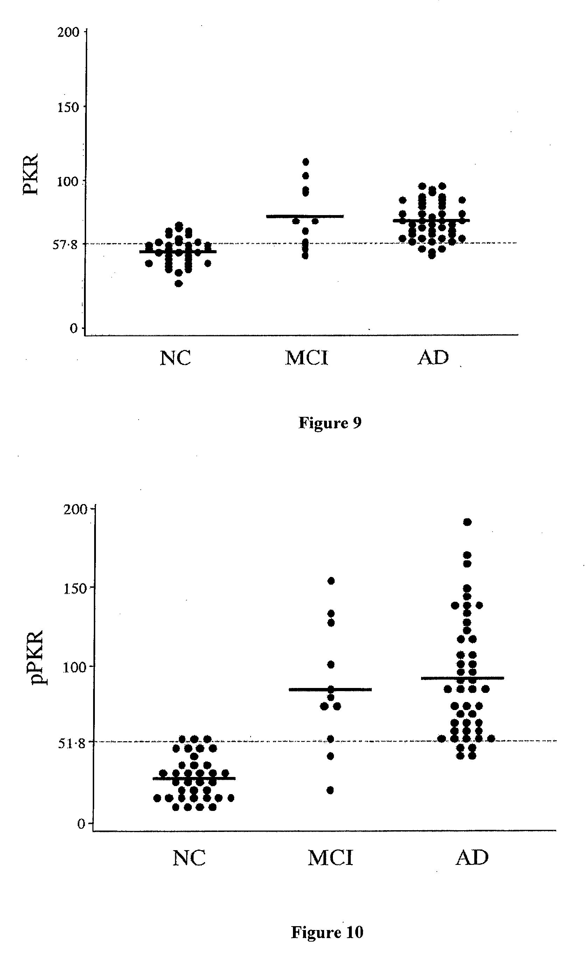

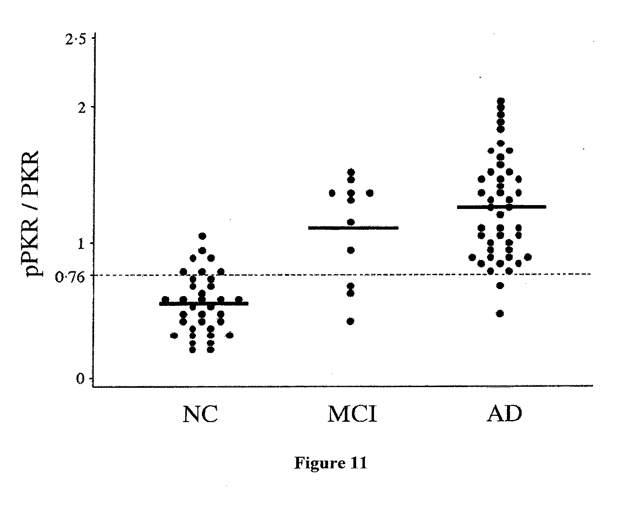

   Les figures 9 à 11 représentent respectivement les niveaux dans le LCR de PKR total et de pPKR446 (en unité de densité optique), ainsi que le rapport pPKR446/PKR total (sans unité), mesurés dans l'Exemple 3, pour des individus témoins (NC), des individus souffrant de troubles cognitifs légers (MCI) amnésiques et des individus souffrant de la maladie d'Alzheimer. Les barres horizontales pleines représentent les valeurs moyennes et les barres horizontales en pointillés la valeur seuil.

### EXEMPLE

### Exemple 1

### 1. Patients et méthodes

### Patients

46 patients diagnostiqués comme ayant la maladie d'Alzheimer (AD) et 39 individus témoins n'ayant pas la maladie d'Alzheimer ont été inclus dans l'étude. Les patients AD ont été inclus sur la base des critères NINCDS-ADRDA. Tous les patients provenaient du Centre Mémoire de l'hôpital Lariboisière (Paris, France). Aucun des individus témoins ne présentait de trouble cognitif et ne remplissait les critères de la maladie d'Alzheimer. Des échantillons de liquide céphalorachidien (LCR) ont été collectés après ponction lombaire dans le cadre de la procédure de diagnostic classique, en association avec un examen neurologique, des tests sanguins de routine, des tests neuropsychologiques et une imagerie IRM du cerveau. L'étude a reçu l'approbation du comité d'éthique de l'Hôpital Paris Nord Bichat.

### Analyse du LCR par marquage de Western : détermination des niveaux de PKR total et PKR phosphorylée sur la thréonine 446 (pPKR446)

Les échantillons de LCR ont été prétraités sur colonne (ProteoExtract™, Calbiochem®) pour retirer l'albumine et les IgG puis préparés, selon les techniques standards, pour un marquage de type Western. Les concentrations de protéines ont été déterminées à l'aide du kit *Micro BCA Protein Assay* (Thermo Scientific) selon les instructions du fabriquant. Les échantillons de LCR ont été séparés sur gel de polyacrylamide 4-12% *NuPAGE BisTris* (Invitrogen) puis électrotransférés sur des membranes de nitrocellulose (GE Healthcare). Les membranes ont été incubées avec des anticorps primaires de lapin reconnaissant respectivement pPKR446 spécifiquement au niveau de la thréonine 446 phosphorylée et la totalité des protéines PKR (SantaCruz), bloquées dans 5% de lait en PBS puis incubées en présence du fluorophore IR Dye™ 800 couplé à un anticorps anti-lapin (Rockland Immunochemical Inc.) dilué au 1/5000. Les protéines liées ont été visualisées à l'aide du système d'imagerie *Odyssey* (LI-COR biosciences) puis quantifiées par densitométrie à l'aide du logiciel *Multigauge* (Fuji). Le rapport pPKR446/PKR total a été déterminé pour chaque patient et chaque individu témoins. Tous les résultats ont été ajustés par rapport à un témoin interne (cerveau humain atteint de la maladie d'Alzheimer). L'analyse statistique de la quantification a été effectuée à l'aide de la version 5 du logiciel *Prism* (GraphPad).

### Analyse du LCR par ELISA : β-amyloïde₁₋₄₂(Aβ42), tau total (T-tau), tau phosphorylé au niveau de la thréonine 181 (ptau 181)

Les niveaux de Aβ42, T-tau and ptau181 ont été déterminés par ELISA *(Innotest tests Abeta, Innotests tau, Innotests p181tau,* Innogenetics, Gand, Belgique) dans les mêmes échantillons de LCR que ceux utilisés pour la détermination des niveaux de PKR total et de pPKR446 en suivant les recommandations du fabriquant.

### Analyse statistique

Les statistiques descriptives ont été utilisées pour décrire les caractéristiques des patients et des témoins. Les groupes ont été comparés en utilisant le test t de Student pour les mesures continues et le test du chi² pour les proportions. Les distributions des niveaux de PKR total et de pPKR446, ainsi que le rapport du niveau de pPKR446 sur le niveau de PKR total (pPKR446/PKR total) au sein des deux groupes (patients et témoins) ont été représentés par la méthode des graphes en boites à moustaches. L'analyse de courbes ROC (*Receiving Operating Characteristics)* a été utilisée pour déterminer les meilleures valeurs de coupure *(cutoff)* du niveau des marqueurs du LCR mesurés et pour calculer l'aire sous les courbes (AUC). Les meilleures valeurs de coupure ont été définies comme donnant la somme de la sensibilité et de la spécificité la plus élevée. Le coefficient de corrélation de Spearman entre PKR et pPKR446 d'une part, et Aβ42, T-tau et ptau d'autre part a également été déterminé.

### 2. Résultats

Les caractéristiques des individus étudiés sont présentées dans le **Tableau 1** suivant :

**Tableau 1 : caractéristiques des individus de l'étude**

| | Témoins | Alzheimer (AD) | |
|---|---|---|---|
| | (N=39) | (N=46) | P |
| Age, en années | 69,5 (10.8) | 70,7 (8,9) | 0,59 |
| Nombre de femmes (%) | 21 (62) | 26 (57) | 0.64 |
| MMSE | 27.1 (2.9) | 19.4 (6.4) | <0.001 |
| | | | |
| Aβ42, en pg/mL | 798.7 (121.6) | 409.8 (149.1) | <0.001 |
| T-tau, en pg/mL | 189.2 (55.1) | 598.0 (328.0) | <0.001 |
| ptau, en pg/mL | 42.5 (11.4) | 107.6 (51.9) | <0.001 |
| | | | |
| PKR total, unités arbitraires | 53.7 (10.6) | 71.9(12.2) | <0.001 |
| pPKR446, unités arbitraires | 30.9 (19.5) | 94.9 (38.5) | <0.001 |
| Rapport pPKR446/PKR | 0,59 (2,47) | 1,28 (3,71) | <0.001 |

| | | | |
|---|---|---|---|
| Sauf pour le nombre de femmes, les valeurs indiquées représentent la moyenne des individus, l'écart type est indiqué entre parenthèses. MMSE : score au test cognitif *Mini Mental Status Examination.* | | | |

Les données du tableau indiquent que les valeurs moyennes des concentrations de PKR et de PKR activée (pPKR446) et du rapport des concentrations pPKR446/PKR sont significativement plus élevées dans le LCR des patients atteints de maladie d'Alzheimer par rapport aux individus témoins.

Ces observations sont confirmées pour les données dans leur ensemble par les représentations en boites à moustache données dans les **Figures 1, 2** **et** **3****.** En particulier, l'ensemble des valeurs obtenues pour les concentrations de pPKR446 et pour le rapport pPKR446/PKR total des individus témoins est significativement inférieur à celui des patients atteints de la maladie d'Alzheimer.

Par ailleurs, les courbes ROC ont été établies pour le PKR total, pPKR446 et le rapport pPKR446/PKR total. Les données de sensibilité et de spécificité tirées de ces courbes sont données dans le **Tableau 2** suivant :

**Tableau 2 : Aire sous la courbe (AUC) des courbes ROC**

| Biomarqueurs | AUC | Seuil | Sensibilité | Spécificité | Correctement criblé |
|---|---|---|---|---|---|
| PKR total | 0,87 | 62 | 76,1 | 79,5 | 77,7 |
| pPKR446 | 0,96 | 40 | 100 | 76,9 | 89,4 |
| pPKR446/PKR total | 0,96 | 77 | 97,8 | 87,2 | 92,9 |
| | | | | | |
| Aβ42 | 0.95 | 584 | 94,4 | 90,9 | 91,9 |
| T-tau | 0.97 | 282 | 93,2 | 100 | 95,2 |
| ptau181 | 0.98 | 61 | 90,9 | 100 | 93,6 |

L'utilisation des niveaux de PKR total et surtout de PKR activé (pPKR446) et du rapport pPKR446/PKR total permet de détecter les malades atteints de la maladie d'Alzheimer. On observe notamment que la détermination du niveau de PKR activé (pPKR446) et du rapport pPKR446/PKR total permet une détection plus sensible de la maladie d'Alzheimer que celle offerte par les marqueurs diagnostics Aβ42, T-tau et ptau181.

Les coefficients de corrélation de Spearman entre les marqueurs Aβ42, T-tau et ptau181 d'une part, et PKR total, pPKR446 et pPKR446/PKR total d'autre part, sont présentés dans le **Tableau 3** suivant et dans la **Figure 7****.**

**Tableau 3 : Coefficients de corrélation de Spearman**

| | Aβ42 | | T-tau | | ptau181 | |
|---|---|---|---|---|---|---|
| | Rho | P | Rho | P | Rho | P |
| PKR total | -0,37 | 0,003 | 0,45 | <0,001 | 0,46 | <0,001 |
| pPKR446 | -0,47 | <0,001 | 0,56 | <0,001 | 0,58 | <0,001 |
| pPKR446/PKR total | -0,47 | <0,001 | 0,56 | <0,001 | 0,57 | <0,001 |

Les coefficients de corrélation de Spearman entre les marqueurs Aβ42, T-tau et ptau181 d'une part, et PKR total, pPKR446 et pPKR446/PKR sont relativement faibles. Ces deux groupes de marqueurs apparaissent donc relativement indépendant les uns des autres, ce qui laisse envisager que la combinaison des marqueurs de l'invention avec Aβ42, T-tau et ptau181 pourrait améliorer l'efficacité diagnostic de ces derniers.

### Exemple 2

En suivant la méthodologie de l'**Exemple** **1,** les inventeurs ont également comparé le rapport du niveau de pPKR446 sur le niveau de PKR total dans le LCR de patients atteints de la maladie d'Alzheimer (AD), sélectionnés comme cela est indiqué dans l'**Exemple** **1**, au même rapport mesuré dans le LCR d'individus témoins sans troubles cognitifs (HC), de témoins présentant des troubles cognitifs sans rapport avec la maladie d'Alzheimer (par exemple secondaires à une dépression ou un autre trouble psychiatrique, à une démence fronto-temporale, à une démence vasculaire ou à une maladie à corps de Levy) (CC), d'individus présentant une démence alcoolique (OH), et d'individu présentant un trouble cognitif léger (MCI) **(****Figure 8****).**

On observe que la valeur moyenne du rapport pPKR446/PKR total est plus élevée dans le LCR de patients atteints de la maladie d'Alzheimer que dans le LCR d'individus témoins ne présentant pas de troubles cognitifs (comme cela a été déjà vu dans l'**Exemple** **1)** ainsi que dans le LCR d'individus présentant une démence alcoolique, dont la valeur moyenne du rapport est d'ailleurs similaire à celle des témoins sans troubles cognitifs. Dans le cas présent, cela indique que l'élévation du rapport pPKR446/PKR total est spécifique de la maladie d'Alzheimer et possiblement plus généralement des démences neurodégénératives.

On observe en outre que la valeur moyenne du rapport pPKR446/PKR total dans le LCR de individus atteints de troubles cognitifs légers (MCI) est intermédiaire par rapport à celle des patients atteints de la maladie d'Alzheimer et à celle des individus témoins sans troubles cognitifs. Dans la mesure où le taux de conversion annuel des individus présentant des MCI en patients souffrant de la maladie d'Alzheimer est d'environ 15%, certains des individus ne développant pas de maladie d'Alzheimer ultérieurement, il est probable que l'élévation du rapport pPKR446/PKR total chez des individus atteints de MCI soit indicative de la présence d'une maladie d'Alzheimer naissante ou à stade précoce (c'est à dire avant l'apparition de symptômes de démence) ou qu'elle soit indicative d'individus à risque de développer la maladie d'Alzheimer à court terme. Par ailleurs, la valeur moyenne du rapport pPKR446/PKR total dans le LCR d'individus témoins présentant des troubles cognitifs sans rapport avec la maladie d'Alzheimer est similaire à celle des témoins sans troubles cognitifs, ce qui indique que l'élévation du rapport pPKR446/PKR total chez les individus atteints de MCI est spécifique de la maladie d'Alzheimer et possiblement plus généralement des démences neurodégénératives

Enfin, on observe qu'alors que certains des patients atteints de la maladie d'Alzheimer présentent des niveaux des marqueurs Aβ42, T-tau et ptau181 dans le LCR normaux (AD-PLn) ou intermédiaires, c'est-à-dire compris entre la limite normale et la limite pathologique (AD-PLi), la valeur du rapport pPKR446/PKR total est élevée, ce qui indique que ce rapport peut être utile pour identifier des patients atteints de la maladie d'Alzheimer qui ne pourraient pas être identifiés à l'aide des marqueurs classiques.

### Exemple 3

Une étude clinique, complémentaire à celle de l'**Exemple 1,** a été mise en oeuvre.

### 1. Patients et méthodes

### Patients

Pendant un an, 119 individus consécutifs venant au Centre Mémoire de l'hôpital Lariboisière (Paris, France) pour une analyse du liquide céphalorachidien (LCR) afin d'étudier des troubles cognitifs ont été inclus dans l'étude. Ont été exclus, du fait de leur faible nombre, 5 individus présentant une démence fronto-temporale ou une maladie à corps de Lewy Body et 6 individus ayant eu un accident vasculaire cérébral, ayant un score supérieur à 4 sur l'échelle d'ischémie d'Hachinski ou ayant une maladie cardiovasculaire avec une conséquence visible en IRM du cerveau (infarctus multiples des gros vaisseaux, infarctus unique placé stratégiquement, ganglions basaux multiples, lacunes de la substance blanche, lésions periventriculaires extensives de substance blanche). Avant le diagnostic, tous les individus ont été soumis à un examen clinique standardisé incluant un historique médical et des examens physiques et neurologiques. Des tests en laboratoire ont été mis en oeuvre pour tous les individus, dont une mesure des taux sériques de cobalamine et de folate, un panel de chimie, des test de fonction de la thyroïde, une sérologie de la syphilis, une mesure des taux de protéine réactive C, une formule sanguine complète, et une IRM du cerveau. Les diagnostics ont été établis par une équipe multidisciplinaire de neurologues et de neuropsychologues spécialisés dans les troubles cognitifs. Le diagnostic de maladies d'Alzheimer a été établi conformément aux critères NINCDS-ADRDA (Mc Khann et al. (1984) Neurology 34:939-44) et les individus souffrant de troubles cognitifs légers (MCI) amnésiques remplissaient les critères habituels (Petersen et al. (2001) Arch. Neurol. 58:1985-92). Cette étude a été approuvée par le comité d'éthique des Centres Hopitalo-Universitaires de Paris (CEERB CHU Bichat, Paris, France). Tous les individus et les soignants ont donné leur accord écrit informé concernant l'analyse du LCR.

### Procédures relatives au LCR

Les ponctions lombaires ont été pratiquées chez les individus à jeun dans le mois suivant le diagnostic Clinique. Le LCR a été collecté en tube de polypropylène de 12 mL. Dans les deux heures, les échantillons de LCR ont été centrifugés à 1800 g pendant 10 min. à 4°C. Une petite quantité de LCR a été utilisée pour les analyses de routine, dont un comptage des cellules totales, un examen bactériologique et les niveaux de glucose et de protéine totaux. Le LCR a été réparti en tube de polypropylène de 500 µL et stocké à -80°C en attente d'analyse. Le niveau dans le LCR de Aβ42, T-tau et ptau181 ont été mesurés à l'aide du test ELISA sandwich *Innotest* selon les instructions du fabricant (Innogenetics, Gant, Belgique). Les critères de positivité des biomarqueurs ont été définis en fonction des anomalies des niveaux de Aβ42, T-tau et ptau181, selon les seuils de coupure utilisés au Centre Mémoire de l'hôpital Lariboisière (Aβ42 < 500 pg/mL; T-tau > 302 pg/mL ; ptau181 > 65 pg/mL). L'ensemble des analyses biologique a été réalisé dans un seul laboratoire. L'équipe de biologistes impliquée dans l'analyse du LCR n'était pas informée du diagnostic clinique. La qualité de l'analyse du LCR a été validée par un consortium européen (programme de contrôle qualité des biomarqueurs du LCR de l'association Alzheimer).

### Détermination des niveaux de PKR total et de pPKR446 dans le LCR

Les échantillons de LCR ont été prétraités sur colonne (ProteoExtract™, Calbiochem®, Darmstadt, Allemagne) pour retirer l'albumine et les IgG, selon les instructions du fabricant. Brièvement, 800 µL du tampon d'équilibrage ont été ajoutés à la colonne pour la réhydrater. Ensuite, 300 µL de chaque échantillon de LCR ont été déposés sur des colonnes individuelles à usage unique et élués par gravité. Les échantillons filtrés ont été obtenus après élution de la colonne par deux lavages avec un tampon de liaison spécifique fourni par le fabricant. Les échantillons débarrassés des IgG et de l'albumine ont été aliquotés et conservés à -20°C. L'efficacité de l'étape de filtration a été évaluée par une analyse d'immunomarquage en utilisant un anticorps dirigé contre l'albumine sérique humaine (Santa Cruz, Danvers, MA, Etats-Unis). Les concentrations de protéines ont été déterminées à l'aide du kit *Micro BCA Protein Assay* (Thermo Scientific, Cergy-Pontoise, France) selon les instructions du fabriquant.

Les échantillons protéiques de LCR ont été séparés sur gel de polyacrylamide 4-12% *NuPAGE BisTris* (Invitrogen) puis électrotransférés sur des membranes de nitrocellulose (GE Healthcare, Chalfont St. Giles, Royaume-Uni) à 400 mA par gel dans 25 mM Tris (pH 8,3), 200 mM glycine et 20% éthanol. Après le transfert, les membranes de nitrocellulose ont été bloquées dans 5% (poids/volume) de lait en TBS, puis incubées avec l'anticorps primaire.

Les anticorps primaires suivants ont été utilisés. Un anticorps de lapin anti-PKR446 (Santa Cruz), un anticorps de lapin anti-PKR (Cell Signaling, Beverly, MA, Etats-Unis), un anticorps de souris anti-Albumine sérique (Santa Cruz). Des IgG anti-souris conjugués au fluorophore IR Dye™ 700DX et des IgG anti-lapin conjugués au fluorophore IR Dye™ (Rockland Immunochemical Inc., Gilbertsville, PA, Etats-Unis) ont été utilisés comme anticorps secondaires. Les protéines liées ont été visualisées à l'aide du système d'imagerie *Odyssey* (LI-COR biosciences, Lincoln, NE, Etats-Unis) puis quantifiées par densitométrie à l'aide du logiciel *Multigauge* (Fuji film, Tokyo, Japon). Les mesures sont exprimées en unités de densité optique (ODU).

Afin d'évaluer la fiabilité des mesure des niveaux de PKR total et de pPKR446, les inventeurs ont mis en oeuvre une étude de test-retest sur le LCR de 25 individus sélectionnés au hasard et calculé le coefficient de corrélation intraclasse entre deux mesures successives des niveaux de PKR total et de pPKR446.

### Analyses statistiques

Les caractéristiques des individus sont présentées selon leur diagnostic clinique (contrôles neurologiques ou témoins (NC), troubles cognitifs légers (MCI) et maladie d'Alzheimer (AD)) et comparées entre les trois groupes à l'aide du test du χ2 pour les variables de catégorie et analyse de variance pour les variables continues. L'analyse de courbes ROC *(Receiving Operating Characteristics)* a été utilisée pour déterminer le pouvoir discriminant de PKR total, pPKR446 et du rapport pPKR446/PKR total pour différencier les individus NC et AD. Les valeurs de seuils de coupures optimales ont été identifiées en maximisant l'indice de Youden (sensibilité + spécificité - 100). La corrélation entre les différents biomarqueurs a été évaluée en utilisant les coefficients de corrélation de Pearson. Toutes les valeurs de p étaient bilatérales et une valeur de P inférieure ou égale à 0,05 a été considérée statistiquement significative. Les analyse statistiques ont été réalisées à l'aide des logiciels SAS version 9.2 (SAS Institute, Cary, Caroline du Nord, Etats-Unis) and Stata version 10.0 (Statacorp LP, College Station, Texas, Etats-Unis).

### 2. Résultats

Parmi les 108 individus inclus, 6 AD, 10 témoins (NC) and 1 MCI ont été exclus pour des raisons techniques liées à l'analyse du LCR (8 du fait d'une quantité insuffisante de LCR et 9 pour une concentration protéique trop faible dans le LCR). Ces individus ne différaient pas des individus inclus en ce qui concerne l'âge et le rapport des sexes. Un total de 91 individus a donc été finalement inclus dans l'étude (AD, n=45 ; MCI amnésique, n=11 ; NC, n=35). Les individus NC ont été dirigés vers le Centre Mémoire de l'hôpital Lariboisière pour des plaintes cognitives (troubles anxio-dépressifs majeurs 14, complication d'attaque vasculaire cérébrale 5, démence alcoolique 4, syndrome d'apnée du sommeil 2, syndrome de Sjôgren 2, sarcoïdose 1, sclérose en plaque 2, sclérose latérale amyotrophique 1, maladie de Lyme 1, hydrocéphalie à pression normale 1, épilepsie 1, neuropathie périphérique 1).

Les caractéristiques cliniques des individus AD, MCI et NC sont présentées dans le **Tableau 4** suivant :

**Tableau 4 : caractéristiques des individus de l'étude**

| | NC | MCI | AD | |
|---|---|---|---|---|
| Caractéristiques | (n=35) | (n=11) | (n=45) | p |
| Age (années) | 64,0 (9,2) | 76,9(10,5) | 70,8 (9,1) | <0,001 |
| Femmes, n (%) | 23 (65,7) | 7 (63,6) | 28 (62,2) | 0,95 |
| MMSE | 25,7 (3,4) | 24,1 (1,7) | 19,6 (6,5) | 0,001 |
| | | | | |
| Biomarqueurs du LCR (pg/mL) | | | | |
| Aβ42 | 808,8 (183,9) | 608,7 (276,4) | 414,7 (150,6) | <0,001 |
| T-tau | 189,6 (59,2) | 316,1 (124,2) | 590,7 (328,7) | <0,001 |
| ptau181 | 45,1 (12,8) | 63,1 (18,8) | 106,3 (52,2) | <0,001 |
| | | | | |
| PKR^{†} dans le LCR | | | | |
| PKR total | 52,2 (9,6) | 75,3 (21,6) | 72,5 (12,6) | <0,001 |
| pPKR446 | 29,0 (13,9) | 85,0 (40,4) | 92,3 (38,0) | <0,001 |
| pPKR446/PKR total | 0,55 (0,23) | 1,10(0,37) | 1,25 (0,38) | <0,001 |

| | | | | |
|---|---|---|---|---|
| Sauf pour le nombre de femmes, les valeurs indiquées représentent la moyenne des individus, l'écart type est indiqué entre parenthèses ; MMSE : score au test cognitif *Mini Mental Status Examination*;^{†} Unités de densité optique | | | | |

L'âge moyen est légèrement plus faible chez les individus NC par rapport aux individus AD. Les biomarqueurs du LCR, Aβ42, T-tau et ptau181, sont significativement différents chez les individus AD et MCI par rapport aux individus NC. La détermination des niveaux de PKR total, de pPKR446 et du rapport pPKR446/PKR total est réalisée dans les mêmes échantillons de LCR pour les trois groupes d'individus. Le niveau de PKR total, et notamment le niveau de pPKR446, sont élevés chez les individus AD.

On observe en particulier qu'il y a une différence statistiquement significative entre les trois groupes en ce qui concerne les niveaux de PKR total et de pPKR446, et le rapport pPKR446/PKR total. Par exemple, le niveau moyen de pPKR est de 29,0 +/- 13,9 chez les individus NC, 85,0 +/- 40,4 chez les individus MCI et 92,3 +/- 38,0 chez les individus ADts (ODU). Ceci est également visible dans les **Figures 9-11** qui présentent les résultats des individus et la valeur seuil déterminée ci-dessous.

Le **Tableau 5** ci-dessous présente les résultats des courbes ROC établies pour le niveau de PKR total, le niveau de pPKR446 et le rapport pPKR446/PKR total concernant la discrimination des individus AD et NC.

**Tableau 5 : Aire sous la courbe (AUC) des courbes ROC**

| Biomarqueurs | AUC (SE) | Valeur seuil | Sensibilité (%) | Spécificité (%) | Youden |
|---|---|---|---|---|---|
| Aβ42 | 0,95 (0,02) | 606 | 96,7 | 88,9 | 0,86 |
| T-tau | 0,95 (0,02) | 282 | 91,1 | 96,7 | 0,88 |
| ptau181 | 0,95 (0,02) | 61 | 88,9 | 90,0 | 0,79 |
| | | | | | |
| PKR total | 0,90 (0,03) | 57,8 | 91,1 | 71,4 | 0,63 |
| pPKR446 | 0,98 (0,01) | 51,8 | 91,1 | 94,3 | 0,85 |
| pPKR446/PKR total | 0,95 (0,02) | 0,76 | 95,6 | 82,9 | 0,79 |

| | | | | | |
|---|---|---|---|---|---|
| SE : Erreur Standard | | | | | |

L'aire sous la courbe (AUC) est de 0,98 pour pPKR446 et 0,95 pour pPKR446/PKR total. Ainsi, une valeur seuil de 51,8 ODU pour pPKR446 permet de distinguer des individus AD d'individus NC, avec une sensibilité de 91,1% et une spécificité de 94,3%. Ces valeurs sont du même ordre que celles déterminées pour Aβ42, T-tau et ptau181 dans les mêmes échantillons de LCR. On constate que la valeur AUC associée à pPKR446 est la meilleure quel que soit le marqueur considéré, y compris les marqueurs du LCR classiques Aβ42, T-tau et ptau181.

Le test-retest, c'est à dire la double détermination des niveaux des marqueurs dans un même échantillon afin d'étudier la fiabilité des mesure, a été mise en oeuvre sur 25 échantillon de LCR (10 NC, 2 MCI et 13 AD). De valeurs fiables des coefficients de corrélation intraclasse entre deux mesures consécutives PKR total et de pPKR446 ont pu être mis en évidence (respectivement 0,94 et 0,97).

En revanche, aucune corrélation n'a pu être établie entre les niveaux de PKR total et de pPKR446n et l'âge ou les scores MMSE. Par ailleurs, on observe que chez les individus AD, le niveau de pPKR446 est plus élevé chez les hommes que chez les femmes.

Les inventeurs ont également étudiés la corrélation entre le niveau de PKR total, le niveau de pPKR446, le rapport pPKR446/PKR total et les niveaux de Aβ42, T-tau et ptau181 chez les individus AD et NC **(Tableau 6).**

**Tableau 6 : Corrélation entre le niveau de PKR total, le niveau de pPKR446, le rapport pPKR446/PKR total et les niveaux de Aβ42, T-tau et ptau181**

| | Coefficient de corrélation de Pearson | | | | | |
|---|---|---|---|---|---|---|
| | Aβ42 | | T-tau | | ptau181 | |
| | r | P | r | P | r | P |
| AD (n=45) | | | | | | |
| PKR total | -0,10 | 0,33 | 0,29 | 0,06 | 0,31 | 0,041 |
| pPKR446 | 0,04 | 0,79 | 0,17 | 0,26 | 0,30 | 0,045 |
| pPKR446/PKR total | 0,14 | 0,36 | 0,05 | 0,73 | 0,19 | 0,21 |
| | | | | | | |

| NC (n=35) | | | | | | |
|---|---|---|---|---|---|---|
| PKR total | -0,12 | 0,51 | 0,17 | 0,36 | 0,20 | 0,40 |
| pPKR446 | -0,02 | 0,90 | 0,27 | 0,15 | 0,04 | 0,83 |
| pPKR446 /PKR total | 0,06 | 0,77 | 0,25 | 0,18 | 0,15 | 0,44 |

On observe que chez les individus AD, les concentrations de ptau181 sont corrélées aux niveaux de PKR total et de pPKR446 mais avec un coefficient de corrélation faible. En revanche, aucune corrélation n'est trouvée entre les niveaux de PKR total ou de pPKR446 et ceux de Aβ42 et de T-tau chez les individus AD. De plus, aucune corrélation n'a pu être mise en évidence entre le niveau de PKR total ou le niveau de pPKR446 et les niveaux de Aβ, T-tau et ptau181 chez les individus NC.

Ceci indique donc que le niveau de PKR total, le niveau de pPKR446, ou le rapport pPKR446 apportent une information essentiellement indépendante par rapport aux marqueurs classiques du LCR, ce qui permet notamment d'anticiper que l'association du niveau de PKR total, du niveau de pPKR446, ou du rapport pPKR446, aux niveaux de Aβ42, T-tau et ptau181 permettrait d'améliorer les performances des tests actuels basés sur ces marqueurs.

Par ailleurs, les inventeurs ont observé que chez les 45 individus AD, 6 présentaient des échantillons de LCR qui ne répondaient pas aux critères de positivité pour les niveaux de Aβ42, T-tau et ptau181, mais qui présentaient pourtant des niveaux augmentés de pPKR446 et du rapport pPKR446/PKR total **(Tableau 7).**

**Tableau 7: détermination du niveau de PKR total, du niveau de pPKR446, et du rapport pPKR446/PKR total chez des individus AD pour lesquels les niveaux de Aβ42, T-tau et ptau181 sont normaux**

| | | | | | Biomarqueurs | | | | | |
|---|---|---|---|---|---|---|---|---|---|---|
| Individus | Sexe | Age | Diagnostic | MMSE | Aβ42 | T-tau | ptau181 | T-PKR | pPKR | pPKR/T-PKR |
| Patient 1 | F | 77,5 | AD | 26 | 647 | 195 | 41 | 49,8 | 73,7 | 1,48 |
| Patient 2 | F | 82,2 | AD | 27 | 802 | 282 | 59 | 57,9 | 55,3 | 0,96 |
| Patient 3 | F | 81,6 | AD | 22 | 865 | 184 | 47 | 53,6 | 63,1 | 1,18 |
| Patient 4 | F | 88,0 | AD | 19 | 641 | 191 | 46 | 85,2 | 92,6 | 1,09 |
| Patient 5 | M | 66,7 | AD | 26 | 279 | 195 | 56 | 84,5 | 141,1 | 1,67 |
| Patient 6 | F | 67,8 | AD | 26 | 751 | 420 | 74 | 68,9 | 135,7 | 1,97 |

| | | | | | | | | | | |
|---|---|---|---|---|---|---|---|---|---|---|
| T-PKR = PKR total ; pPKR = pPKR446 | | | | | | | | | | |

Ainsi, l'évaluation de PKR total et de pPKR446 peut mettre en évidence une anomalie, alors que les biomarqueurs classiques n'en montrent pas, ce qui renforce leur intérêt et confirme que les biomarqueurs de l'invention apportent une information essentiellement indépendante de celle des biomarqueurs classiques.

Finalement, les inventeurs ont comparé les niveaux des différent biomarqueurs chez les individus MCI **(Tableau 8).**

**Tableau 8 : détermination du niveau de PKR total, du niveau de pPKR446, et du rapport pPKR446/PKR total chez des individus MCI amnésiques**

| | | | | | Biomarqueurs | | | | | |
|---|---|---|---|---|---|---|---|---|---|---|
| Individus | Sexe | Age | MCI | MMSE | Aβ42 | T-tau | p181tau | T-PKR | pPKR | pPKR/T-PKR |
| Patient 1 | F | 76·7 | stable | 26 | 924 | 399 | 85 | 90,9 | 131,6 | 1,45 |
| Patient 2 | F | 82·1 | Converti | 22 | 664 | 206 | 35 | 55,9 | 72,4 | 1,29 |
| Patient 3 | F | 85·3 | Stable | 24 | 312 | 288 | 59 | 113,2 | 72,6 | 0,64 |
| Patient 4 | M | 82·4 | Converti | 26 | 870 | 328 | 79·5 | 64,5 | 43,5 | 0,67 |
| Patient 5 | F | 79·5 | Converti | 26 | 447 | 270 | 44 | 102,5 | 154,9 | 1,51 |
| Patient 6 | F | 86·5 | Stable | 24 | 265 | 602 | 89 | 94,0 | 126,6 | 1,34 |
| Patient 7 | F | 66·9 | stable | 24 | 854 | 253 | 64 | 53,3 | 51,4 | 0,97 |
| Patient 8 | M | 49·9 | stable | 23 | 309 | 109 | 36 | 49,9 | 20,5 | 0,41 |
| Patient 9 | F | 79·6 | stable | 27 | 332 | 357 | 58 | 72,4 | 98,8 | 1,36 |
| Patient 10 | M | 74·5 | Converti | 22 | 795 | 358 | 74 | 59,4 | 79,6 | 1,34 |
| Patient 11 | M | 83·2 | Converti | 23 | 924 | 307 | 71 | 72,2 | 82,8 | 1,15 |

| | | | | | | | | | | |
|---|---|---|---|---|---|---|---|---|---|---|
| T-PKR = PKR total ; pPKR = pPKR446 ; Stable indique une absence de passage vers la maladie d'Alzheimer sur la durée de l'étude ; Converti indique un passage vers la maladie d'Alzheimer sur la durée de l'étude | | | | | | | | | | |

On observe que pour une valeur de coupure de 51,8 ODU concernant le niveau de pPKR446, 81,8 % des individus MCI amnésiques présentaient un niveau de pPKR446 anormal. Parmi les cinq individus qui déclarèrent la maladie d'Alzheimer sur la durée de l'étude qui dura 1 an, un seul des individus présentait des niveaux de PKR total et de pPKR446 normaux. Par comparaison, un seul des cinq individus présentait un niveau de Aβ42 anormal et seuls trois des cinq individus présentaient un niveau anormal de T-tau et de ptau181. Il apparait donc que les biomarqueurs de l'invention pourraient être plus sensibles que Aβ42, T-tau et ptau181 pour identifier parmi les individus MCI ceux qui sont à un stade précoce, asymptomatique, de la maladie d'Alzheimer, ou ceux qui sont à risque de développer à court terme la maladie d'Alzheimer.

Par ailleurs, parmi les six patients MCI stables, quatre présentaient des niveaux de PKR total et de pPKR446 anormaux. Une étude sur une plus longue période permettra de d'évaluer si ces individus déclarent effectivement la maladie d'Alzheimer.

## Revendications

1. Méthode de *diagnostic in vitro* d'une maladie neurodégénérative chez un individu, dans laquelle :
- on détermine le niveau de la protéine kinase ARN double-brin dépendante (PKR) dans un échantillon de liquide céphalorachidien de l'individu,
- on en déduit si l'individu souffre d'une maladie neurodégénérative.

2. Méthode selon la revendication 1, dans laquelle la maladie neurodégénérative est sélectionnée dans le groupe constitué de la maladie d'Alzheimer, de la maladie de Huntington, de la maladie de Creutzfeld-Jakob, et de la maladie de Parkinson.

3. Méthode selon la revendication 1 ou 2, dans laquelle la maladie neurodégénérative est la maladie d'Alzheimer.

4. Méthode selon l'une des revendications 1 à 3, dans laquelle l'individu n'est pas atteint de démence.

5. Méthode selon l'une des revendications 1 à 4, dans laquelle l'individu souffre de troubles cognitifs légers (MCI).

6. Méthode selon l'une des revendications 1 à 5, dans laquelle le niveau, dans un échantillon de liquide céphalorachidien, d'au moins un marqueur biologique de maladie neurodégénérative est normal.

7. Méthode selon la revendication 6, dans laquelle le marqueur biologique est choisi dans le groupe constitué du marqueur β-amyloïde (Aβ), de tau total (T-tau), et de tau phosphorylé (ptau).

8. Méthode selon l'une des revendications 1 à 7, dans laquelle on déduit que l'individu souffre d'une maladie neurodégénérative à partir du niveau de PKR phosphorylé dans l'échantillon, du niveau de PKR total dans l'échantillon, ou du rapport du niveau de PKR phosphorylé dans l'échantillon sur le niveau de PKR total dans l'échantillon.

9. Méthode selon la revendication 1 à 8, dans laquelle on déduit que l'individu souffre d'une maladie neurodégénérative à partir de la comparaison entre :
- le niveau de PKR phosphorylé dans l'échantillon, le niveau de PKR total dans l'échantillon, ou le rapport du niveau de PKR phosphorylé dans l'échantillon sur le niveau de PKR total dans l'échantillon, et
- au moins une valeur prédéfinie.

10. Méthode selon l'une des revendications 1 à 9, comprenant en outre la détermination du niveau d'au moins un autre marqueur biologique de maladie neurodégénérative tel que défini dans les revendications 6 ou 7.

## Patentansprüche

1. *In-vitro*-Diagnoseverfahren für eine neurodegenerative Erkrankung bei einem Individuum, bei dem:
- der Gehalt der Doppelstrang-RNA-abhängigen Proteinkinase (PKR) in einer Probe von Zerebrospinalflüssigkeit des Individuums bestimmt wird,
- davon abgeleitet wird, ob das Individuum an einer neurodegenerativen Erkrankung leidet.

2. Verfahren nach Anspruch 1, wobei die neurodegenerative Erkrankung ausgewählt ist aus der Gruppe bestehend aus Alzheimer-Krankheit, Huntington-Krankheit, Creutzfeld-Jakob-Krankheit und Parkinson-Krankheit.

3. Verfahren nach Anspruch 1 oder 2, wobei die neurodegenerative Erkrankung Alzheimer-Krankheit ist.

4. Verfahren nach einem der Ansprüche 1 bis 3, wobei das Individuum nicht an Demenz leidet.

5. Verfahren nach einem der Ansprüche 1 bis 4, wobei das Individuum an leichter kognitiver Beeinträchtigung (MCI) leidet.

6. Verfahren nach einem der Ansprüche 1 bis 5, wobei der Gehalt wenigstens eines biologischen Markers für neurodegenerative Erkrankungen in einer Probe von Zerebrospinalflüssigkeit normal ist.

7. Verfahren nach Anspruch 6, wobei der biologische Marker ausgewählt ist aus der Gruppe bestehend aus β-Amyloid-Marker (Aβ), Gesamt-Tau (T-tau) und phosphoryliertem Tau (ptau).

8. Verfahren nach einem der Ansprüche 1 bis 7, wobei auf Basis des Gehalts von phosphorylierter PKR in der Probe, des Gehalts von Gesamt-PKR in der Probe oder des Verhältnisses des Gehalts von phosphorylierter PKR in der Probe zu Gehalt von Gesamt-PKR in der Probe abgeleitet wird, dass das Individuum an einer neurodegerierativen Erkrankung leidet.

9. Verfahren nach Anspruch 1 bis 8, wobei auf Basis des Vergleichs zwischen:
- dem Gehalt von phosphorylierter PKR in der Probe, dem Gehalt von Gesamt-PKR in der Probe oder dem Verhältnis des Gehalts von phosphorylierter PKR in der Probe zu Gehalt von Gesamt-PKR in der Probe, und
- wenigstens einem vordefinierten Wert
abgeleitet wird, dass das Individuum an einer neurodegenerativen Erkrankung leidet.

10. Verfahren nach einem der Ansprüche 1 bis 9, umfassend weiterhin die Bestimmung des Gehalts wenigstens eines weiteren biologischen Markers für neurodegenerative Erkrankungen gemäß den Ansprüchen 6 oder 7.

## Claims

1. Method for the *in vitro* diagnosis of a neurodegenerative disease in an individual, in which:
- the level of double-stranded RNA-dependent protein kinase (PKR) in a sample of the individual's cerebrospinal fluid is determined,
- it is deduced whether the individual suffers from a neurodegenerative disease.

2. A method according to claim 1, in which the neurodegenerative disease is selected from the group comprising Alzheimer's disease, Huntingdon's disease, Creutzfeldt-Jakob disease and Parkinson's disease.

3. A method according to claim 1 or 2, in which the neurodegenerative disease is Alzheimer's disease.

4. A method according to any one of claims 1 to 3, in which the individual is unaffected by dementia.

5. A method according to any one of claims 1 to 4, in which the individual suffers from mild cognitive disturbances (MCD).

6. A method according to any one of claims 1 to 5, in which the level of at least one biological marker of neurodegenerative disease in a sample of cerebrospinal fluid is normal.

7. A method according to claim 6, in which the biological marker is selected from the group comprising the β-amyloid (Aβ), total tau (T-tau), and phosphorylated tau (ptau) markers.

8. A method according to any one of claims 1 to 7, in which it is deduced that an individual suffers from a neurodegenerative disease from the level of phosphorylated PKR in the sample, the level of total PKR in the sample, or the ratio between the level of phosphorylated PKR in the sample and the level of total PKR in the sample.

9. A method according to any one of claims 1 to 8, in which it is deduced that an individual suffers from a neurodegenerative disease on the basis of a comparison between:
- the level of phosphorylated PKR in the sample, the level of total PKR in the sample, or the ratio between the level of phosphorylated PKR in the sample and the level of total PKR in the sample, and
- at least one predefined value.

10. A method according to any one of claims 1 to 9, further comprising determination of the level of at least one other biological marker of a neurodegenerative disease as defined in claims 6 or 7.
